(19) Europäisches Patentamt / European Patent Office / Office européen des brevets

(11) **EP 2 987 470 A1**

(12) **EUROPEAN PATENT APPLICATION**
published in accordance with Art. 153(4) EPC

(43) Date of publication:
**24.02.2016 Bulletin 2016/08**

(51) Int Cl.:
**A61F 2/915** (2013.01)

(21) Application number: **14785097.8**

(86) International application number:
**PCT/JP2014/060702**

(22) Date of filing: **15.04.2014**

(87) International publication number:
**WO 2014/171447 (23.10.2014 Gazette 2014/43)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **18.04.2013 JP 2013087781**

(71) Applicant: **Piolax Medical Devices, Inc.**
**Yokohama-shi**
**Kanagawa 240-0023 (JP)**

(72) Inventors:
• **YAMAUCHI, Kiyoshi**
**Sendai-shi**
**Miyagi 981-3211 (JP)**

• **UEGAKI, Michihiro**
**Sendai-shi**
**Miyagi 980-8579 (JP)**
• **TOYOKAWA, Yoshihide**
**Yokohama-shi**
**Kanagawa 240-0023 (JP)**

(74) Representative: **Grünecker Patent- und Rechtsanwälte**
**PartG mbB**
**Leopoldstraße 4**
**80802 München (DE)**

(54) **STENT AND PROCESS FOR PRODUCING SAME**

(57) Provided are a stent which has sufficient strength, is easily reduced in diameter to facilitate insertion of the stent into a tube, and can be smoothly expanded from inside the tube and a process for producing the stent. This stent (10) is a self-expandable stent constituted of an Ni-Ti base alloy or Co-Cr base alloy and formed into a cylindrical shape with mesh openings. The stent has been configured so that the Af point is 22-26°C, the stress-displacement curve has a yield point, and the crystal grains in a cross-section of the stent have an average cross-sectional area, as determined by the area fraction method, of 0.2-50 $\mu m^2$. The stent can be produced by inserting a core bar (22) into a cylindrical stent base (20), forming mesh openings with laser light, subsequently removing the core bar from the stent base, expanding the stent base to a given diameter in an atmosphere having a temperature of 350°C or lower, and then subjecting the expanded stent base to a heat treatment at 400-600°C for 5-60 minutes to regulate the Af point to 22 to 26°C.

*FIG. 1*

EP 2 987 470 A1

Printed by Jouve, 75001 PARIS (FR)

**Description**

**TECHNICAL FIELD**

The present invention relates to a stent which is placed in a tubular organ, for example, a bile duct, a ureter, a trachea, a blood vessel, etc., or other body tissue and to a method for producing the same.

**BACKGROUND ART**

There have hitherto been performed treatments using a stent, for example, a treatment in which the stent is placed in a stenosed portion or an occluded portion in a tubular organ, such as a bile duct, a ureter, a trachea, a blood vessel, etc., so as to dilate the tubular organ, thereby making bile, blood, or the like easy to flow therethrough, a treatment in which the stent is placed in a portion where an aneurysm has occurred, so as to prevent the aneurysm from rupturing, or the like.

On the occasion of placing a stent, for example, the stent is diametrically contracted, received in a tube, such as a sheath, a catheter, etc., conveyed into a target position of a body tissue, such as a bile duct, etc., and then released from the tube, thereby undergoing self-expansion, or a balloon disposed in the inside of the stent is diametrically expanded, whereby the stent is placed in a predetermined portion.

A stent constituted of an Ni-Ti-based alloy is, for example, produced in the following way. That is, a metal tube constituted of an Ni-T-based alloy is heat treated at a predetermined temperature to achieve straightening and then processed with a laser light, thereby forming plural mesh-shaped openings. Thereafter, an expansion treatment of the stent is carried out plural times under an atmosphere higher than the heat treatment temperature in the above-described straightening step, for example, at 400 to 500°C, until the stent reaches a predetermined outer diameter, whereby the stent having a predetermined diameter is produced.

However, in the above-described production method, the heat treatment for straightening of the tube, the expansion treatment to be carried out plural times, and the like are carried out under a high-temperature atmosphere over a relatively long time, and hence, the strength of the stent may be lowered due to coarsening of crystal grains, progress of recrystallization, or the like, resulting in breakage or the like.

In order to solve the above-described problem, such as breakage, etc., for example, Patent Literature 1 as described below describes a method for producing a highly elastic stent including the steps of inserting a core metal into a tube-shaped stent base body; maintaining, as needed, linearity of the stent base; subsequently, cutting slot forming portions with a laser light while suppressing a heat effect by the laser light on the periphery of the slot forming portions so as to form slots and thus to make a stent; and expanding the stent from which the core metal has been removed to a predetermined diameter while performing a heat treatment at 350°C or lower.

The stent produced by the above-described production method has such properties that a load increases with displacement without exhibiting a distinct yield on a load-displacement curve obtained by a compression test and a bending test, an Af point thereof is ordinary temperature or lower, and the stent exhibits superelasticity at ordinary temperature. Thus, a problem, such as breakage, etc., is hardly caused.

**CITATION LIST**

**PATENT LITERATURE**

**Patent Literature 1:** WO-2012-008579-A

**SUMMARY OF INVENTION**

**TECHNICAL PROBLEM**

However, the stent produced by the production method described in Patent Literature 1 becomes superelastic at ordinary temperature so that it does not undergo plastic deformation. Thus, there is encountered such inconvenience that when it is intended to diametrically contract the stent in order to receive the stent in a tube, such as a catheter, etc., the stent is hardly contracted, so that it is hardly received in the tube. In addition, the stent received in a diametrically-contracted state in the tube is liable to expand due to its own superelastic force; however, the stent is liable to be reversely smashed due to a repulsive force from the inner periphery of the tube and fatigued. Thus, when the stent is released from the tube, the stent may not smoothly expand.

In consequence, an object of the present invention is to provide a stent which has sufficient strength, is readily diametrically contracted so that it can be readily received in a tube, and when releasing from the inside of the tube, can be smoothly expanded, and a method for producing the same.

## SOLUTION TO PROBLEM

In order to achieve the foregoing object, the stent according to the present invention is a self-expandable stent including an Ni-Ti-based alloy or a Co-Cr-based alloy and formed in a cylindrical shape having mesh-shaped openings, wherein the stent has an Af point of 22 to 26°C and has a yield point on a stress-displacement curve, and crystal grains in a cross section of the stent have an average cross-sectional area, as determined by the area fraction method, of 0.2 to 50 $\mu m^2$.

The method for producing a stent according to the present invention includes inserting a core metal into a cylindrical stent base constituted of an Ni-Ti-based alloy or a Co-Cr-based alloy, forming mesh-shaped openings with a laser light, subsequently removing the core metal from the stent base, expanding the stent base to a predetermined diameter under an atmosphere at 350°C or lower, and then heat treating the resulting stent base at 400 to 600°C for 5 to 60 minutes so as to adjust an Af point to 22 to 26°C.

In the method for producing a stent according to the present invention, it is preferred that the opening forming step with a laser light is carried out in such a manner that water is jetted onto the stent base to form a water column, and the laser light is irradiated on the stent base while reflecting the laser light in this water column, thereby forming mesh-shaped openings in the stent base.

In the method for producing a stent according to the present invention, it is preferred that the heat treatment step after the expansion treatment of the stent base is carried out at 450 to 550°C for 10 to 40 minutes.

In the method for producing a stent according to the present invention, it is preferred that the heat treatment step is carried out only one time after the expansion treatment of the stent base.

## ADVANTAGEOUS EFFECTS OF INVENTION

In accordance with the stent according to the present invention, the stent has an Af point of 22 to 26°C and has a yield point is present on a stress-displacement curve, and hence, it does not become superelastic at ordinary temperature. Furthermore, when the stent is diametrically contracted, it is readily maintained in a diametrically-contracted state thereof, so that the stent can be relatively readily received in a tube, such as a catheter, a sheath, etc.

The crystal grains in a cross section of the stent have an average cross-sectional area, as determined by the area fraction method, of 0.2 to 50 $\mu m^2$, and hence, the strength of the stent can be increased. As a result, even in a state where the stent is received in the tube, it is possible to make the stent hard to fatigue. When the stent is released from the tube, the stent can be smoothly expanded.

In accordance with the method for producing a stent according to the present invention, the temperature when the stent base is expanded is a relatively low temperature as 350°C or lower, and the subsequent heat treatment is carried out at 400 to 600°C; and hence, coarsening of crystal grains or recrystallization, or the like of the tissue can be suppressed, and it is possible to produce a high-strength stent with fine crystal grains, which has an Af point of 22 to 26°C and has a yield point on a stress-displacement curve, and in which the crystal grains in a cross section thereof have an average cross-sectional area, as determined by the area fraction method, of 0.2 to 50 $\mu m^2$.

## BRIEF DESCRIPTION OF DRAWINGS

Fig. 1 is a perspective view showing an embodiment of a stent according to the present invention.
Fig. 2A is a development view of the stent. Fig. 2B is a development view of a stent in other example.
Figs. 3A and 3D show a method for producing a stent according to the present invention. Fig. 3A is an explanatory view showing a step of inserting a core metal into a stent base. Fig. 3B is an explanatory view showing a drawing processing step. Fig. 3C is an explanatory view showing a straightening step. Fig. 3D is an explanatory view showing an opening forming step with a laser light.
Figs. 4A and 4B show a method for producing a stent according to the present invention. Fig. 4A is an explanatory view showing a core metal-removing step. Fig. 4B is an explanatory view of a state where the core metal has been removed from the stent base.
Figs. 5A to 5C shows a method for producing a stent according to the present invention. Fig. 5A is an explanatory view showing an expanding step of a stent base. Fig. 5B is an explanatory view showing a shape memory treatment step after the expansion treatment of the stent base and a subsequent heat treatment step. Fig. 5C is an explanatory view showing a step of drawing an expansion device from the stent base after the heat treatment.
Fig. 6 is a stress-displacement curve graph of each of Example and Comparative Example.

Figs. 7A and 7B show an orientation mapping (IPF) image by the electron backscatter diffraction (EBSD) method. Fig. 7A corresponds to Example. Fig. 7B corresponds to Comparative Example.

Fig. 8 is an explanatory view of the area fraction method used for the measurement of an average cross-sectional area of crystal grains.

## DESCRIPTION OF EMBODIMENTS

An embodiment of the stent according to the present invention is hereunder explained by reference to the accompanying drawings.

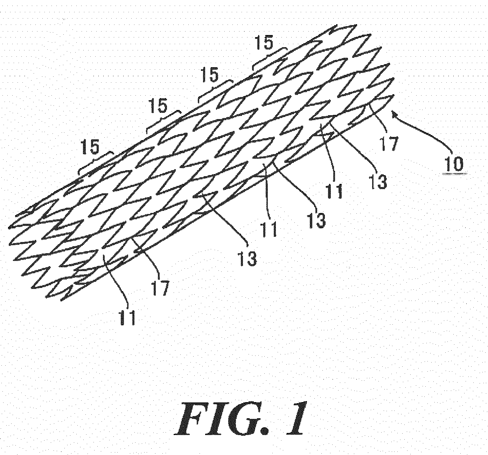

As shown in Fig. 1, a stent 10 of this embodiment is formed in a cylindrical shape having plural mesh-shaped openings 11 and is of a self-expandable type such that when an external force is not applied, it is in a diametrically-expanded state.

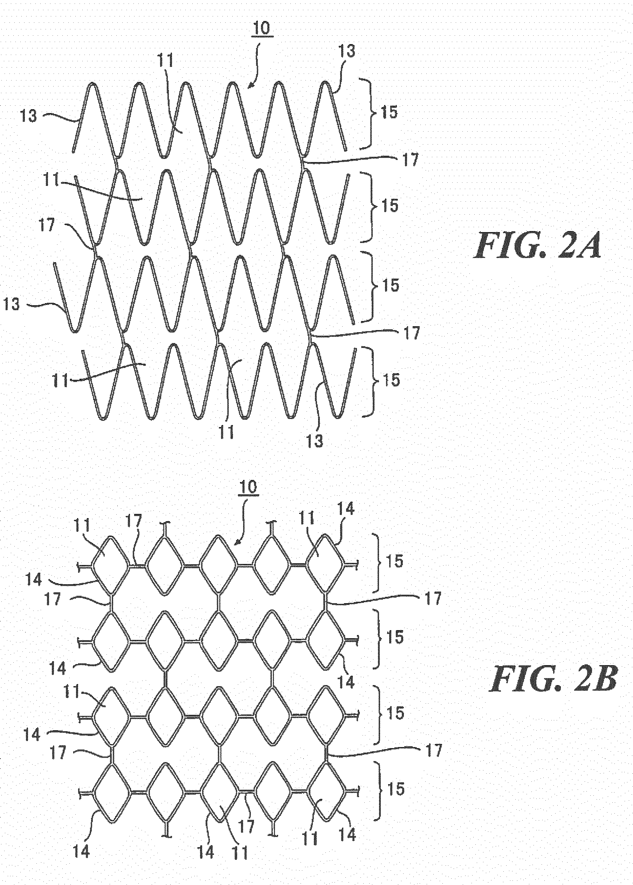

Explanation is made while also referring to the development view of Fig. 2A. This stent 10 is molded in a cylindrical shape having mesh-shaped openings by processing a metal cylinder with a laser light. In this embodiment, a pattern having mesh-shaped openings of the stent 10 is formed as follows. That is, circumferential units 15 each extending in a zigzag shape along the circumferential direction, in which both ends of this zigzag-shaped portion 13 are annularly connected to each other, are formed. Bent portions of the zigzag-shaped portion 13 of each of the circumferential units 15 are connected to each other through a connection portion 17. According to this, the plural circumferential units 15 are connected to each other in the axial direction through the connection portions 17, whereby the stent 10 is formed in a cylindrical shape as a whole.

As other example of a pattern having mesh-shaped openings of the stent 10, as shown in Fig. 2B, the stent 10 may be one in which plural frame-shaped bodies 14 each having the opening 11 are connected to each other in the circumferential direction to form the circumferential units 15, and these are connected to each other in the axial direction through the plural connection portions 17, thereby forming a cylinder shape. The shape and disposition pattern of the openings 11 of the stent 10 are not limited to those described in the foregoing Figs. 2A and 2B, and the shape and disposition pattern are not particularly limited so long as it is diametrically contractable and expandable.

A cover member constituted of, for example, polyurethane, silicone, natural rubber, a nylon elastomer, a polyether block amide, polyethylene, polyvinyl chloride, *vinyl acetate*, a fluorine-based resin, or the like, may be disposed in the inside and/or outside of the stent 10.

As a material of the stent 10, an Ni-Ti-based alloy, such as Ni-Ti, Ni-Ti-Co, Ni-Ti-Cu, Ni-Ti-Fe, Ni-Ti-Nb, Ni-Ti-V, Ni-Ti-Cr, Ni-Ti-Mn, etc., or a Co-Cr-based alloy, such as Co-Cr, Co-Cr-Mo, Co-Cr-Ni, etc., is used.

Then, this stent 10 has an Af point of 22 to 26°C. The "Af point" as referred to herein means a temperature at which the austenite transformation finishes in a shape memory alloy, such as an Ni-Ti-based alloy, a *C-Cr-based alloy*, etc., and when the temperature becomes this temperature or higher, the resulting alloy returns to a shape memorized by the shape memory treatment. When the Af point is lower than 22°C, an elastic force of the stent 10 is high at a temperature at which the stent 10 is generally used, for example, at a temperature in an operating room or the like, so that it does not hardly contracted, and it becomes difficult to receive the stent 10 in a tube, such as a sheath, a catheter, etc. On the other hand, in the case where the Af point exceeds 26°C, when the stent 10 is placed in a tubular organ or a body tissue, the stent 10 hardly returns to the memorized diametrically-expanded shape, so that the usefulness is lowered.

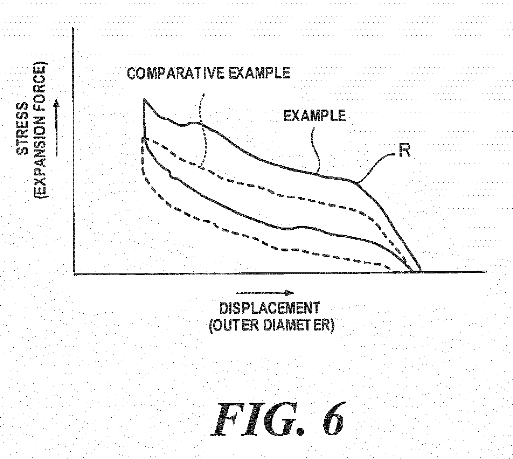

This stent 10 has a yield point on its stress-displacement curve. That is, when using a stent radial expansion force equipment, "RX550", manufactured by Machine Solutions Inc., the stent is diametrically contracted at 1 mm/min in the diametrically contracting direction equally over the entirety of the stent until the outer diameter of the stent reaches 2.5 mm, and then diametrically expanded at 1 mm/min in the diametrically expanding direction until the outer diameter of the stent reaches the initial value, a yield point (a portion shown by R in the figure) can be distinctly grasped on a stress (expansion force of the stent) vs. displacement (outer diameter displacement of the stent) curve (see Fig. 6).

In this stent 10, crystal grains in a cross section have an average cross-sectional area, as determined by the area fraction method, of 0.2 to 50 $\mu m^2$, and preferably 0.5 to 30 $\mu m^2$.

When the average cross-sectional area of the crystal grains is less than 0.2 $\mu m^2$, the stent is increased in strength, whereas it is insufficient in flexibility; and hence, when the stent 10 is diametrically contracted and received in the tube, and then released from the tube, the resulting stent hardly expands. On the other hand, when the average cross-sectional area of the crystal grains exceeds 50 $\mu m^2$, the strength of the stent cannot be sufficiently increased, and when the stent 10 is received in a diametrically-contracted state in the tube, the resulting stent becomes readily fatigued.

The average cross-sectional area of crystal grains refers to an average cross-sectional area of crystal grains when a boundary at which an orientation angle difference is 5° or more is defined as a crystal grain boundary in an IPF map using the known electron backscatter diffraction method (EBSD method) with a scanning electron microscope (SEM).

In this case, the average cross-sectional area of crystal grains is measured by the area fraction method. That is, when an area of the entire measuring tissue in an IPF map is defined as 100, and areas of the crystal grains in the IPF map are defined as S1, S2, S3, S4, ..., the average cross-sectional area of crystal grains is expressed by the following formula.

$$\text{Average cross-sectional area of crystal grains} = (S1 \times S1/100) + (S2 \times S2/100) + (S3 \times S3/100) + (S4 \times S4/100) + ...$$

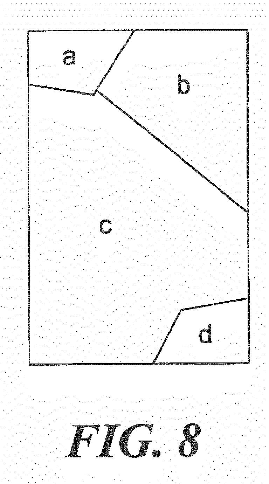

For example, as shown in Fig. 8, when an area of a crystal grain a is defined as 8, an area of a crystal grain b is defined as 25, an area of a crystal grain c is defined as 59, and an area of a crystal grain d is defined as 8, an average cross-sectional area of the crystal grains becomes $(8 \times 0.08 + 25 \times 0.25 + 59 \times 0.59 + 8 \times 0.08 = 42.34)$.

The IPF map and the average cross-sectional area of crystal grains by the EBSD method can be, for example, measured by installing an EBSD apparatus (electron diffraction crystal orientation analysis apparatus, "HIKARI", manufactured by TSL Solutions Inc.) in SEM ("JSM-7800F", manufactured by JEOL Ltd.) and using an exclusive software (OIM Analysis 6.2).

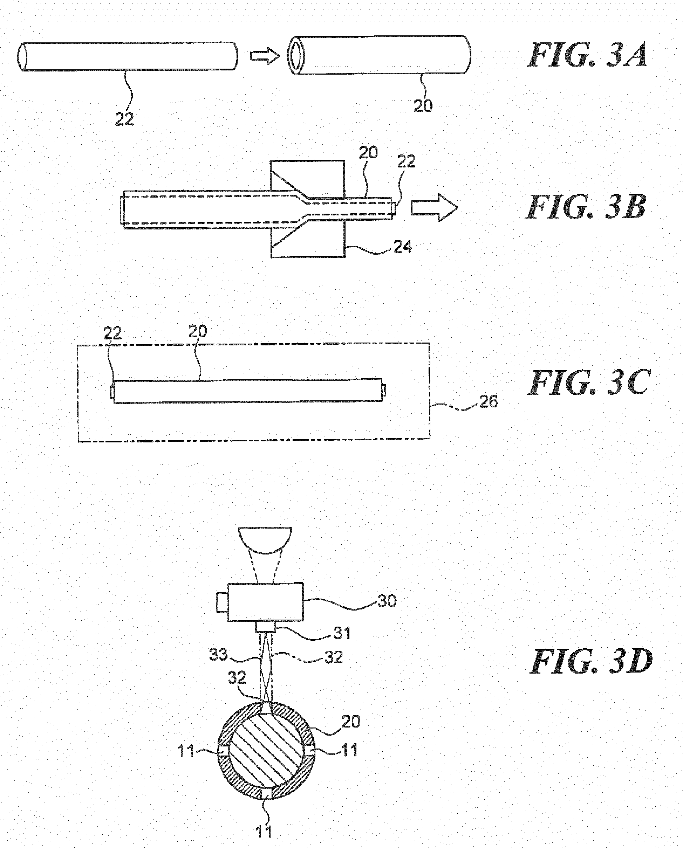

Next, an embodiment of the method for producing a step according to the present invention is explained by reference to Figs. 3A to 5C.

This production method includes inserting a core metal into a cylindrical stent base constituted of an Ni-Ti-based alloy or a Co-Cr-based alloy, forming mesh-shaped openings with a laser light, subsequently removing the core metal from the stent base, expanding the stent base to a predetermined diameter under an atmosphere at 350°C or lower, and then heat treating the resulting stent base at 400 to 600°C for 5 to 60 minutes so as to adjust an Af point to 22 to 26°C.

First of all, as shown in Fig. 3A, a core metal 22 is inserted into a cylindrical stent base 20 constituted of an Ni-Ti-based alloy or a Co-Cr-based alloy formed of the above-described material.

Thereafter, as shown in Fig. 3B, the stent base 20 having the core metal 22 inserted thereinto is inserted into a hole of a die 24 having a smaller diameter than the stent base 20, and the resultant is subjected to drawing processing or extrusion processing at a predetermined rate, thereby diametrically contracting the stent base 20 to a predetermined diameter.

At that time, a processing rate of the stent base 20 is preferably 10% or more, more preferably 35% or more, and still more preferably 45% or more. When the processing rate of stent base 20 is less than 10%, the tissue which has been processed and hardened by a shape memory treatment or heat treatment after the diametrically contracting processing readily vanishes, and hence, the strength of the stent becomes low.

Subsequently, as shown in Fig. 3C, the stent base 20 is disposed in a heat treatment furnace 26 and held at a predetermined temperature for a predetermined time, thereby straightening the diametrically-contracted stent base 20. The treatment temperature is preferably 400 to 600°C, and more preferably 450 to 550°C; and the holding time is preferably 5 to 60 minutes, and more preferably 20 to 40 minutes. This straightening treatment is carried out, as needed and is not an essential step in the method for producing a stent according to the present invention.

Thereafter, as shown in Fig. 3D, the mesh-shaped openings 11 are formed in the stent base 20. In this embodiment, predetermined portions of the stent base 20 are cut off in a predetermined shape by a so-called water laser, thereby forming the openings 11. Specifically, pressurized water with high pressure is jetted toward the stent base 20 from a nozzle 31 of a laser cutter 30, thereby forming a water column 32. Simultaneously, a laser light 33 injected from the nozzle 31 is irradiated on the stent body 20 while reflecting laser light 33 within the water column 32, thereby forming the openings 11 having a predetermined shape.

As such a water laser cutter, for example, "AQL1900", manufactured by Shibuya Kogyo Co., Ltd., or the like can be used.

As described previously, the openings 11 can be formed with the laser light 33 in the predetermined portions of the stent base 20 while cooling within the water column 32. Thus, a heat effect due to reflection or scattering, or the like of the laser light 33 against the stent base 20 is hardly given, and coarsening of crystal grains or recrystallization, or the like of the tissue is suppressed, so that it is possible to maintain the strength of the stent base 20. Even in a state where the core metal 22 is inserted, the openings 11 can be surely formed in predetermined portions of the stent base 20.

Thereafter, the stent base 20 having mesh-shaped openings 11 is cut in a predetermined length by the laser light 33 or other cutting means.

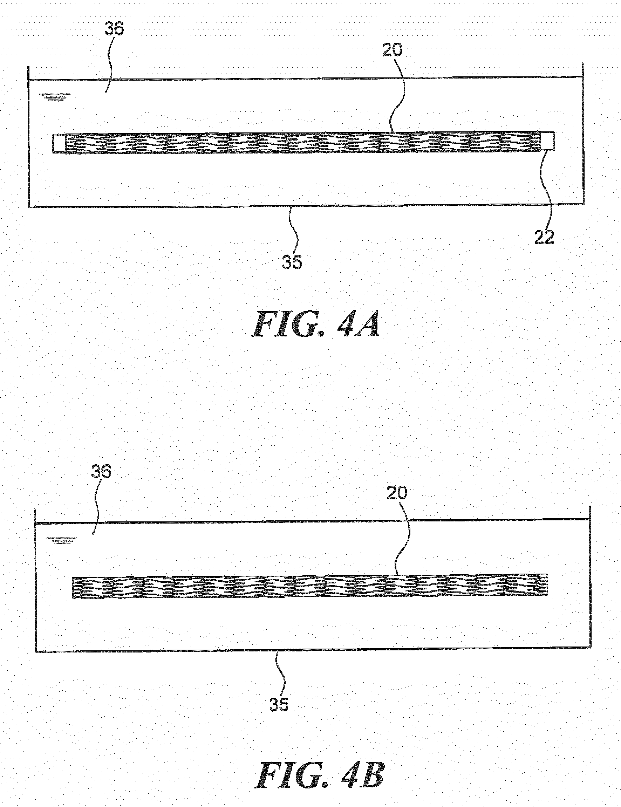

After the foregoing steps, as shown in Fig. 4A, the stent base 20 having the core metal 22 inserted thereinto is dipped in a treatment tank 35 in which a treatment liquid 36, such as nitric acid, etc., is stored, thereby dissolving the core metal 22 therein, as shown in Fig. 4B.

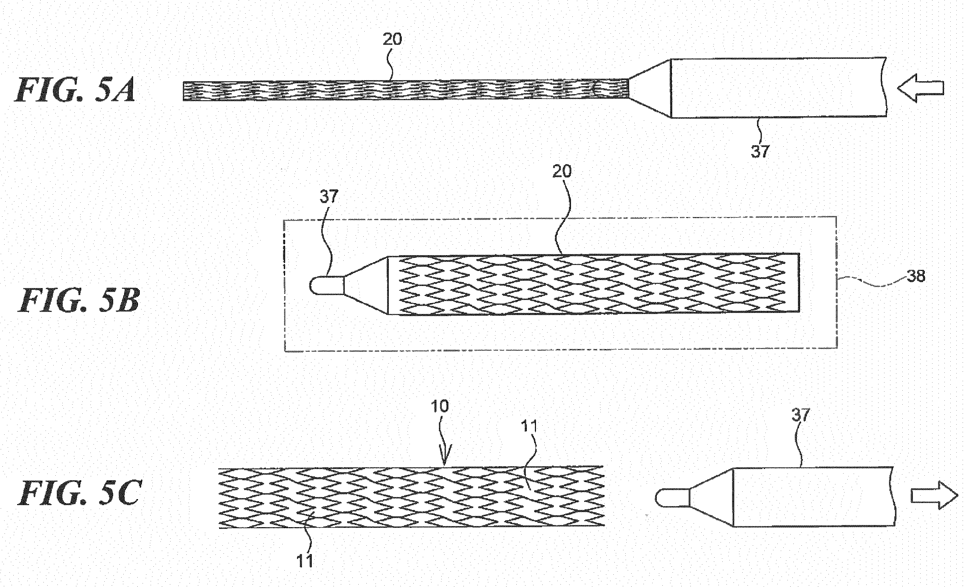

Subsequently, as shown in Fig. 5A, by inserting a distal end portion of an expansion device 37. In the expansion device 37, the distal end portion is diametrically contracted, whereas a base portion thereof is diametrically expanded, from one end of the stent base 20 in the axial direction, the stent base 20 is expanded in diameter and installed in the outer periphery of the expansion device 37.

In this state, as shown in Fig. 5B, the stent base 20 is disposed together with the expansion device 37 in a heat treatment furnace 38 and held under an atmosphere at 350°C or lower for 1 to 60 minutes, thereby subjecting the stent base 20 to a shape memory treatment for the purpose of memorizing the diametrically-expanded state. On this occasion, the temperature is more preferably 300°C or lower. More preferably, the holding time at the time of the shape memory treatment may be 1 to 70 minutes. When the above-described temperature is 350°C or higher, coarsening of crystal grains or recrystallization, or the like of the stent tissue is advanced, resulting in a lowering of the strength.

The stent base 20 is disposed together with the expansion device 37 in the same heat treatment furnace 38 or a separate heat treatment furnace and held at 400 to 600°C for 5 to 60 minutes, thereby subjecting the stent base 20 to a heat treatment such that the Af point reaches 22 to 26°C (see Fig. 5B).

The temperature at the time of the above-described heat treatment is preferably 450 to 550°C, and the holding time is preferably 10 to 40 minutes. By selecting this heat treatment condition, it is possible to produce a stent with good quality having higher strength.

When the temperature at the time of the above-described heat treatment is lower than 400°C, it becomes difficult to set the Af point of the stent to 22 to 26°C, whereas when the temperature exceeds 600°C, coarsening of crystal grains or recrystallization, or the like of the stent tissue is advanced, resulting in a lowering of the strength. When the holding time at the time of the heat treatment is less than 5 minutes, it becomes difficult to uniformly subject the entirety of the stent to the heat treatment, whereas when it exceeds 60 minutes, coarsening of crystal grains or recrystallization, or the like of the stent tissue is advanced, resulting in a lowering of the strength.

It is preferred that the heat treatment step is carried out only one time after the expansion treatment of the stent base. According to this, a heat history of the stent base 20 becomes small; and hence, the processed and hardened tissue of the stent becomes easy to retain, and coarsening of crystal grains or recrystallization, or the like of the stent tissue can be effectively suppressed, so that a stent having higher strength can be obtained.

Thereafter, after the stent base 20 is subjected to furnace cooling in the heat treatment furnace 38 or is discharged from the heat treatment furnace and then subjected to air cooling or quenching, or immediately after the above-described heat treatment, the expansion device 37 is drawn out from the stent base 20 (see Fig. 5C), whereby the stent 10 shown in Fig. 1 can be obtained.

In consequence, according to this production method, the temperature at the time of expanding the stent base is a relatively low temperature as 350°C or lower, and the temperature of the subsequent heat treatment is 400 to 600°C. Thus, coarsening of crystal grains or recrystallization, or the like of the tissue can be suppressed, and it is possible to produce the stent 10 which has an Af point of 22 to 26°C and has a yield point on a stress-displacement curve, and in which crystal grains in a cross section thereof have an average cross-sectional area, as determined by the area fraction method, of 0.2 to 50 $\mu m^2$, so as to miniaturize the crystal grains and to have high strength.

The average cross-sectional area of crystal grains in the cross section of the stent can be made small by decreasing the temperature of the above-described heat treatment or shortening the holding time thereof, or decreasing the number of times of the heat treatment; whereas the average cross-sectional area of crystal grains in the cross section of the stent can be made large by increasing the temperature of the above-described heat treatment or prolonging the holding time thereof, or increasing the number of times of the heat treatment.

Next, the operation and effect of the stent 10 produced by the foregoing production method are explained.

That is, this stent 10 has an Af point of 22 to 26°C and has a yield point on a stress-displacement curve, and hence, it does not become superelastic at ordinary temperature, and when the stent 10 is diametrically contracted, it is readily maintained in a diametrically-contracted state thereof. As a result, when the stent 10 is diametrically contracted and received in a tube, such as a catheter, a sheath, etc., for the purpose of placing the stent 10 in a tubular organ, for example, a bile duct, a ureter, a trachea, a blood vessel, etc., or other body tissue, the stent 10 can be received while maintaining the diametrically contracted state thereof, and hence, the stent 10 can be readily received, and receiving workability can be enhanced. In addition, the average cross-sectional area of crystal grains in the cross section of the stent 10 as determined by the area fraction method is 0.2 to 50 $\mu m^2$. Thus, the crystal grains are miniaturized, the strength of the stent 10 can be increased, it is possible to make the stent hard to fatigue, and when the stent 10 is released from the tube, the stent 10 can be smoothly expanded.

**EXAMPLES**

**(Fabrication of Example)**

An Ni-Ti alloy ingot containing 56% of Ni and 43.8% of Ti, with the balance being inevitable impurities, is processed in a columnar shape; this is molded into the cylindrical stent base 20 having an outer diameter of 5 mm and a length of 1,000 mm by means of mechanical processing (see Fig. 3A); and the core metal 22 is inserted into this stent base 20 and then subjected to drawing processing, thereby forming the stent base 20 having an outer diameter of 3.23 mm at a processing rate of 35% (see Fig. 3B). Thereafter, the plural openings 11 are formed in the stent base 20 by using the laser cutter 30 (see Fig. 3D). Subsequently, the stent base 20 is dipped in the treatment tank 35 so as to dissolve the core metal 22 therein (see Figs. 4A and 4B); the expansion device 37 having an outer diameter of 10 mm is inserted into the stent base 20 (see Fig. 5A); and the stent base 20 is disposed in the heat treatment furnace 38 and subjected to a shape memory treatment at 300°C for 5 minutes (see Fig. 5B). The stent base 20 is subjected to a heat treatment at 500°C for 35 minutes in the heat treatment furnace 38 (see Fig. 5B); and thereafter, the expansion device 37 is drawn out from the stent base 20, thereby fabricating the stent 10 of the Example (see Fig. 5C). The stent of this Example has an outer diameter of 10.5 mm and a length of 10 mm and has an Af point of 24°C.

**(Fabrication of Comparative Example)**

A cylindrical base made of an Ni-Ti alloy ingot is straightened by means of heat reforming at 400°C for 60 minutes; plural openings are then formed by using a YAG laser apparatus; thereafter, the resultant is (1) diametrically expanded at 420°C for 30 minutes to an extent of 4 mm, (2) diametrically expanded at 450°C for 30 minutes to an extent of 7 mm, and (3) diametrically expanded at 500°C for 30 minutes to an extent of 10 mm, respectively by using three dies having a different size from each other; and subsequently, the resulting stent base is subjected to a shape memory treatment at 550°C for 60 minutes, thereby fabricating a stent of the Comparative Example. Other conditions are identical with those of the foregoing Example. The stent of this Comparative Example has an outer diameter of 10.1 mm and a length of 10 mm and has an Af point of 24°C.

**(Preparation of IPF map by the EBSD method and measurement of average cross-sectional area of crystal grains)**

With respect to each of the stents of the foregoing Example and Comparative Example, an IPF map is prepared by an EBSD method using an exclusive software (OIM Analysis 6.2) in an EBSD apparatus (electron diffraction crystal orientation analysis apparatus, "HIKARI", manufactured by TSL Solutions Inc.) installed in SEM ("JSM-7800F", manufactured by JEOL Ltd.), and an average cross-sectional area of crystal grains is measured on the basis of this IPF map by the area fraction method.

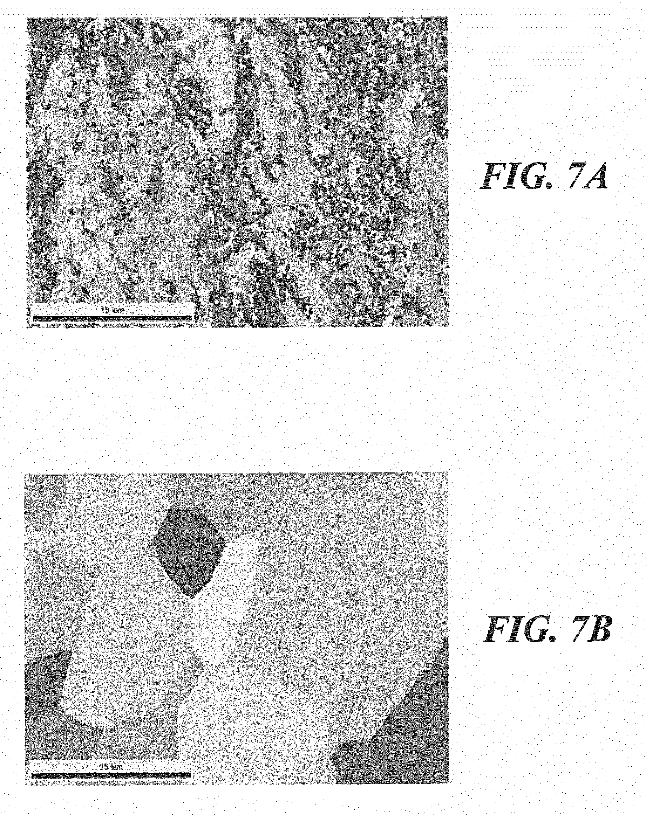

Fig. 7A shows an IPF map of the stent of the Example, and Fig. 7B shows an IPF map of the stent of the Comparative Example. The scales in Figs. 7A and 7B are 15 $\mu$m. As shown in these Figs. 7A and 7B, in the stent of the Comparative Example, the crystal grains are extremely large, whereas in the stent of the Example, the crystal grains are miniaturized. In addition, the average cross-sectional area of crystal grains of the stent of the Example is 2.64332 $\mu$m$^2$ (standard deviation: 0.647377), whereas the average cross-sectional area of crystal grains of the stent of the Comparative Example is 141.769 $\mu$m$^2$ (standard deviation: 54.4368).

**(Measurement of strength)**

With respect to each of the stents of the foregoing Example and Comparative Example, by using a stent radial expansion force equipment, "RX550", manufactured by Machine Solutions Inc., the stent is diametrically contracted at 1 mm/min in the diametrically contracting direction equally over the entirety of the stent until the outer diameter of the stent reached 2.5 mm, and then diametrically expanded at 1 mm/min in the diametrically expanding direction until the outer diameter of the stent reached the initial value, and at that time, a relation between an expansion force of the stent and an outer diameter displacement of the stent (stress-displacement curve) is measured. The results are shown in Fig. 6. As shown in Fig. 6, the stent of the Example is higher in the expansion force and higher in the strength than the stent of the Comparative Example.

**REFERENCE SIGN LIST**

10: Stent
11: Opening
20: Stent base

**Claims**

1. A self-expandable stent including an Ni-Ti-based alloy or a Co-Cr-based alloy and formed in a cylindrical shape having mesh-shaped openings,
   wherein the stent has an Af point of 22 to 26°C and has a yield point on a stress-displacement curve, and crystal grains in a cross section of the stent have an average cross-sectional area, as determined by the area fraction method, of 0.2 to 50 $\mu$m$^2$.

2. A method for producing a stent including
   inserting a core metal into a cylindrical stent base constituted of an Ni-Ti-based alloy or a Co-Cr-based alloy, forming mesh-shaped openings with a laser light, subsequently removing the core metal from the stent base,
   expanding the stent base to a predetermined diameter under an atmosphere at 350°C or lower, and then heat treating the resulting stent base at 400 to 600°C for 5 to 60 minutes so as to adjust an Af point to 22 to 26°C.

3. The method for producing a stent of Claim 2,
   wherein the opening forming step with a laser light is carried out in such a manner that water is jetted onto the stent base to form a water column, and the laser light is irradiated on the stent base while reflecting the laser light in this water column, thereby forming mesh-shaped openings in the stent base.

4. The method for producing a stent of Claim 2 or 3,
   wherein the heat treatment step after the expansion treatment of the stent base is carried out at 450 to 550°C for 10 to 40 minutes.

5. The method for producing a stent of any one of Claims 2 to 4,
   wherein the heat treatment step is carried out only one time after the expansion treatment of the stent base.

**FIG. 1**

*FIG. 2A*

*FIG. 2B*

*FIG. 3A*

*FIG. 3B*

*FIG. 3C*

*FIG. 3D*

**FIG. 4A**

**FIG. 4B**

FIG. 5A

FIG. 5B

FIG. 5C

EP 2 987 470 A1

FIG. 6

*FIG. 7A*

*FIG. 7B*

FIG. 8

## INTERNATIONAL SEARCH REPORT

| International application No. |
| --- |
| PCT/JP2014/060702 |

A. CLASSIFICATION OF SUBJECT MATTER
*A61F2/915*(2013.01)i

According to International Patent Classification (IPC) or to both national classification and IPC

B. FIELDS SEARCHED

Minimum documentation searched (classification system followed by classification symbols)
A61F2/915

Documentation searched other than minimum documentation to the extent that such documents are included in the fields searched
Jitsuyo Shinan Koho          1922–1996    Jitsuyo Shinan Toroku Koho    1996–2014
Kokai Jitsuyo Shinan Koho    1971–2014    Toroku Jitsuyo Shinan Koho    1994–2014

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| Y | JP 2006-141555 A (President of National Cardiovascular Center), 08 June 2006 (08.06.2006), paragraphs [0034], [0037]; fig. 4 to 5 (Family: none) | 1-5 |
| Y | JP 2010-106361 A (Nippon Seisen Co., Ltd.), 13 May 2010 (13.05.2010), paragraphs [0039] to [0043] & WO 2010/038730 A1    & CN 102066595 A | 1 |
| Y | JP 2008-184643 A (Nippon Seisen Co., Ltd.), 14 August 2008 (14.08.2008), paragraphs [0024], [0026], [0027] (Family: none) | 1 |

☒ Further documents are listed in the continuation of Box C.          ☐ See patent family annex.

| | |
| --- | --- |
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search<br>07 July, 2014 (07.07.14) | Date of mailing of the international search report<br>22 July, 2014 (22.07.14) |
| --- | --- |
| Name and mailing address of the ISA/<br>Japanese Patent Office | Authorized officer |
| Facsimile No. | Telephone No. |

Form PCT/ISA/210 (second sheet) (July 2009)

**INTERNATIONAL SEARCH REPORT**

International application No.

PCT/JP2014/060702

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 2012-502190 A  (Fort Wayne Metals Research Products Corp.), 26 January 2012 (26.01.2012), claim 24; paragraph [0037]; fig. 30 & US 2010/0075168 A1    & WO 2010/033873 A1 | 1 |
| Y | WO 2012/008579 A1  (Tohoku University), 19 January 2012 (19.01.2012), paragraphs [0026] to [0065]; claims 1 to 5; fig. 1 to 12 & US 2013/0204391 A1    & EP 2594232 A1 | 2-5 |
| Y | JP 2005-534371 A  (Unison Therapeutics, Inc.), 17 November 2005 (17.11.2005), paragraph [0040] & US 2004/0024444 A1    & WO 2004/010902 A1 | 2-5 |
| Y | US 2013/0067907 A1  (Joel M.Greene), 21 March 2013 (21.03.2013), paragraphs [0001], [0003] & WO 2013/040317 A1 | 2-5 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2012008579 A